Europäisches Patentamt

European Patent Office

Office européen des brevets

(11) Veröffentlichungsnummer: **0 292 810 B1**

## EUROPÄISCHE PATENTSCHRIFT

(45) Veröffentlichungstag der Patentschrift: **11.08.93**

(21) Anmeldenummer: **88107704.4**

(22) Anmeldetag: **13.05.88**

(51) Int. Cl.⁵: **G01N 33/543**, G01N 33/68, //G01N33/569,G01N33/576

(54) Einschritt-Immuntest zur Bestimmung von antigen-spezifischen Antikörpern aus einer der Immunglobulin-Klassen A, M, D oder E und dazu geeignetes Mittel.

(30) Priorität: **23.05.87 DE 3717401**

(43) Veröffentlichungstag der Anmeldung: **30.11.88 Patentblatt 88/48**

(45) Bekanntmachung des Hinweises auf die Patenterteilung: **11.08.93 Patentblatt 93/32**

(84) Benannte Vertragsstaaten: **AT BE CH DE ES FR GB IT LI LU NL SE**

(56) Entgegenhaltungen:
**EP-A- 0 008 473        EP-A- 0 083 869
EP-A- 0 107 551        EP-A- 0 163 312
EP-A- 0 261 493        BE-A- 901 567
FR-A- 2 365 800        GB-A- 2 026 691
US-A- 4 663 277**

(73) Patentinhaber: **BEHRINGWERKE Aktiengesellschaft
Postfach 1140
W-3550 Marburg 1(DE)**

(72) Erfinder: **Ziegelmaier, Robert, Dr.
Hohe Leuchte 33
W-3550 Marburg(DE)**

(74) Vertreter: **Fischer, Hans-Jürgen, Dr. et al
Hoechst AG, Zentrale Patentabteilung, Gebäude F 821
W-6230 Frankturt am Main 80 (DE)**

**Beschreibung**

Die Erfindung betrifft ein immunchemisches Verfahren zum Nachweis und zur Bestimmung von Antikörpern, die für ein bestimmtes Antigen spezifisch sind, aus einer der Immunglobulin-Klassen. Dieses Verfahren ist zum hochsensitiven und spezifischen Nachweis und zur Bestimmung von Antikörpern aus einer der Immunglobulin-Klassen A, M, D oder E geeignet.

Immunglobuline sind Antikörper, die vom Immunsystem des Organismus gegen Fremdstoffe (Antigene) gebildet werden (z.B. Proteine von Krankheitserregern, bakterielle Polysaccharide, Serumproteine, Gewebeproteine oder andere Immunglobuline). Das Immunglobulinmolekül besteht aus einem oder mehreren Sätzen von 4 Polypeptidketten, zwei schweren Ketten mit einem Molekulargewicht von je etwa 53.000 Dalton und zwei leichten Ketten von je etwa 22.000 Dalton, die durch Disulfidbrücken verbunden sind.

Immunglobuline werden im allgemeinen in die Klassen G, A, M, D und E eingeteilt und entsprechend mit IgG, IgA, IgM, IgD und IgE bezeichnet. Diese 5 Immunglobulinklassen unterscheiden sich in den antigenen Determinanten der schweren Kette, die als gamma-, alpha-, my-, delta- und epsilon-Kette bezeichnet werden; darüberhinaus gibt es bei IgG, IgA und IgM auch Immunglobulin-Unterklassen.

Immunglobuline können in Fragmente gespalten werden, welche die antigenbindende Eigenschaft behalten haben, oder in Fragmente ohne antigenbindende Eigenschaft. Antigenbindende Fragmente sind z.B. Fab-, Fab'- und F(ab')$_2$-Fragmente. Fragmente ohne antigenbindende Eigenschaft sind z.B. Fc und Fc'-Fragmente.

Die Konzentrationen an Immunglobulinen im normalen menschlichen Serum sind (in mg/ml): IgG 8-16, IgA 1.4-4, IgM 0.5-2, IgD 0.0-0.4 und IgE 0.000017-0.00045.

Quantitativ sind in humanem Serum Immunglobuline der IgG-Klasse am stärksten vertreten. Immunglobuline der IgM-Klasse treten sehr früh nach einer Infektion auf, weshalb ihre Bestimmung zur Früherkennung einer Infektionskrankheit oder für die Diagnose einer akuten Infektion bedeutsam ist.

Die Immunglobuline der Klasse IgA sind das am zweitstärksten vertretene Immunglobulin und stellen den wichtigsten sekretorischen Antikörper dar.

Immunglobuline der Klassen IgD und IgE können bei bestimmten pathologischen Prozessen in erhöhter Konzentration gefunden werden; IgE beispielsweise hat mastzellensensibilisierende Eigenschaften und spielt bei einer Reihe allergischer Reaktionen eine pathogenetisch bedeutsame Rolle. IgD-Antikörper werden bei Autoimmun-Erkrankungen gefunden.

Die Bestimmung von antigenspezifischen Immunglobulinen, insbesondere einer bestimmten Klasse, ist von besonderer Bedeutung für den Nachweis bestimmter Erkrankungen durch Parasiten, Bakterien oder Viren, wobei hierbei akute von ausgeheilten Infektionen unterscheidbar und gegebenenfalls prognostische Aussagen möglich sind.

Zur Bestimmung von Immunglobulinen sind eine Vielzahl immunologischer Methoden bekannt. Methoden zur physikalischen Trennung von Immunglobulinen nach Klassen, z.B. Immundiffusion, Immunelektrophorese oder Dichtegradienten-Zentrifugation, sind aufwendig, ungenau und störanfällig.

Antigenspezifische Immunglobuline können im sogenannten direkten Verfahren mit Immunoassay-Techniken bestimmt werden; dabei wird eine Immunkomponente mit bindender Affinität für die zu bestimmende Antikörperklasse an einen festen Träger gekoppelt, z.B. Antikörper gegen die μ-Kette von humanem IgM und der antigenspezifische Immunglobulin-Anteil entweder durch markiertes Antigen detektiert oder als Kombination von unmarkiertem Antigen und antigenspezifischem markiertem Antikörper. Quantifiziert wird der an die Festphase gebundene Anteil der markierten Immunkomponente, der der Konzentration des nachzuweisenden Antikörpers direkt proportional ist.

Zur Markierung werden beispielsweise fluoreszierende und chromophore Stoffe oder radioaktive Isotope, Enzyme oder mit Immunkomponenten beladene Partikel wie Erythrozyten oder Latexpartikel verwendet; zur Sichtbarmachung der abgelaufenen Reaktion kann auch eine biologische Funktion des verwendeten Antigens verwendet werden, z.B. die Haemolyse.

Ein Nachteil der Verfahren des Standes der Technik besteht darin, daß der nicht-antigenspezifische Immunglobulinanteil einer angenommenen Immunglobulinklasse mit dem antigenspezifischen Anteil in Konkurrenz um den jeweiligen Festphasenantikörper tritt (Kompetition). Dadurch kann es in Abhängigkeit von dieser Mengenrelation zu unterschiedlichen Ergebnissen kommen, selbst wenn der antigenspezifische Antikörperanteil unverändert gleich ist (der nicht-antigenspezifische Immunglobulinanteil kann dabei um Faktor 5 oder mehr variieren).

In allen bisher beschriebenen und zitierten sog. direkten und indirekten Methoden ist es essentiell, nach Umsetzung (Inkubation) der Probe mit der Immunkomponente am festen Träger und vor Umsetzung mit der Nachweis-Immunkomponente ungebundenes Material durch Waschung zu entfernen. Diese Verfahren werden deshalb als "Zweischritt-Verfahren" bezeichnet (siehe z.B. EP-A-0 008 473).

Dadurch erfordert ein Test mit einer fertigen trägergebundenen Komponente wenigstens drei Reaktionsschritte (Probe/zweite Immunkomponente/Nachweisreaktion), die durch wenigstens 2 Waschschritte voneinander getrennt sind, wobei jeder Reaktionsschritt für sich eine gewisse Reaktionszeit in Anspruch nimmt, so daß sich in der Summe daraus die gesamte Testzeit ergibt.

Die Aufgabe bestand nun darin, den direkten Test zu verkürzen und zu vereinfachen und die Kompetition von nicht-antigenspezifischen gegen antigen-spezifische Immunglobuline auszuschalten, um eine verläßliche quantitative Bestimmung des antigenspezifischen Antikörperanteils zu ermöglichen.

Überraschend wurde nun gefunden, daß dies möglich ist, indem trägergebundene Immunkomponente, analythaltige Probe und markierte Nachweis-Immunkomponente zusammen gebracht werden, ohne zwischen der Zugabe der Probe und der Zugabe der markierten Nachweisimmunkomponente zu waschen.

Dieses "Einschritt-Verfahren" wurde möglich, nachdem es gelungen war zwei Störmöglichkeiten zu beseitigen:

Erstens muß der Einfluß antigen-spezifischer IgG-Antikörper beseitigt werden, so daß sie nicht mehr oder nur noch unwesentlich mit dem Antigen reagieren, wodurch das eigentliche Nachweisverfahren gestört würde. Diese Störung ist deshalb prinzipiell gegeben, weil bei der Bestimmung von antigen-spezifischen Antikörpern aus einer der Immunglobulinklassen IgA, IgM, IgE oder IgD in der Patientenprobe in der Regel antigen-spezifische IgG-Antikörper gleichzeitig und im allgemeinen in einer höheren Konzentration vorhanden sind.

Zweitens ist die Aktivität von Rheumafaktoren (RF), das heißt Antikörper gegen IgG, die verschiedenen Immunglobulinklassen angehören, zu unterbinden, da diese zu einer Verfälschung des Ergebnisses führen kann. Diese Verfälschung ist möglich, weil RF an den Festphasenantikörper gebunden werden und über das vom RF seinerseits gebundene antigen-spezifische IgG Antigen gebunden und damit eine falsch-positive Nachweisreaktion erhalten wird.

Beide Störmöglichkeiten konnten beispielsweise ausgeschaltet werden durch Zugabe von Anti-Human-IgG, gamma-Kette, ("RF-Adsorbens" der Behringwerke AG) zur Probe (zum Beispiel Serum).

Gegenstand der Erfindung ist ein immunchemisches Verfahren zur Bestimmung von Antikörpern, die für ein Antigen spezifisch sind, aus einer der Immunglobulin-Klassen A, M, D oder E in einer Flüssigkeit, wobei eine Festphase, an die Antikörper gegen diese Immunglobulin-Klasse oder ein Fragment eines solchen Antikörpers gebunden sind, mit dieser Flüssigkeit in Kontakt gebracht wird, wodurch das Immunglobulin dieser Klasse an diese Festphase gebunden wird, und diese Festphase entweder mit dem ein Markierungsmittel tragenden Antigen oder mit einem unmarkierten Antigen und einem markierten Antikörper oder einem markierten Fragment eines Antikörpers gegen das Antigen, in Kontakt gebracht wird und aus der Menge des an die Festphase gebundenen Markierungsmittels die Menge dieser Antikörper, die für ein Antigen spezifisch sind, aus einer der Immunglobulin-Klassen bestimmt wird, dadurch gekennzeichnet, daß die Festphase gleichzeitig mit der die zu bestimmenden Antikörper enthaltenden Flüssigkeit und dem entweder markierten oder unmarkierten Antigen in Kontakt ist, wobei als Stoff, der verhindert, daß Immunglobulin G an die Festphase und gegebenenfalls Antigen an IgG gebunden wird, Anti-Human-IgG, aggregiertes humanes oder tierisches IgG oder ein gamma Fc-Fragment zugesetzt wird.

Diese Stoffe können zur Verstärkung des Effektes auch in Kombination verwendet werden.

Ein solcher Stoff, vorzugsweise Anti-Human-IgG, gamma-Kette, (RF-Adsorbens der Behringwerke AG) kann beispielsweise dem Probenverdünnungspuffer zugesetzt werden, vorzugsweise in einer Menge, daß im Mittel 15 mg/ml IgG im Serum (bezogen auf die unverdünnte Probe) komplexiert wird.

Durch diese Maßnahme wird ein Einschritt-Verfahren möglich, wobei die Probe in einer um einen Faktor 3 bis 8 höheren Verdünnung getestet werden kann (zum Beispiel 1:700) im Vergleich zum Zwei-Schritt-Verfahren des Standes der Technik (Testverdünnung 1:100 bis 1:200).

Gleichzeitig wird die erwähnte Kompetition praktisch ausgeschaltet, wodurch es möglich wurde korrekt und reproduzierbar zu quantifizieren und eine höhere Nachweisempfindlichkeit zu erreichen (s. Tabelle 2).

Bevorzugt ist eine Ausführungsform des erfindungsgemäßen Verfahrens, worin markiertes Antigen verwendet wird.

Es kann aber auch unmarkiertes Antigen und ein gegen dieses Antigen gerichteter markierter Antikörper oder ein markiertes Fragment eines solchen Antikörpers verwendet werden.

Zur Markierung werden beispielsweise fluoreszierende und chromophore Stoffe oder radioaktive Isotope, Enzyme oder mit Immunkomponenten beladene Partikel wie Erythrozyten oder Latexpartikel verwendet; zur Sichtbarmachung der abgelaufenen Reaktion kann auch eine biologische Funktion des verwendeten Antigens verwendet werden, z. B. die Haemolyse.

Vorzugsweise wird ein Enzym verwendet.

Bevorzugt werden an die Festphase Antikörper gegen die Immunglobulin-Klasse M oder Fragmente solcher Antikörper, welche die Reaktivität mit diesen Immunglobulinen behalten haben, gebunden.

Vorzugsweise wird das erfindungsgemäße Verfahren zur Bestimmung von Antikörpern verwendet, die gegen Hepatitis B-core Protein, gegen Antigene von Hepatitis A-, Human Immunodeficiency- (HIV), Rötelnoder Cytomegalie-Virus oder Antigene von Treponema pallidum oder Toxoplasma gondii gerichtet sind.

Gegenstand der Erfindung ist auch ein Mittel zur Ausführung des erfindungsgemäßen Verfahrens, das mindestens aus einem Träger, an den für eine der humanen Immunglobulin-Klassen spezifische Antikörper gebunden sind, markiertem Antigen, für das dieses Immunglobulin spezifisch ist, Reagenzien zum Nachweis oder zur Bestimmung der Markierung besteht und als Stoff, der verhindert, daß Immunglobulin G an die Festphase und gegebenenfalls Antigen an Immunglobulin G gebunden, Anti-Human IgG, aggregiertes humanes oder tierisches IgG oder ein gamma-Fc-Fragment.

Vorzugsweise besteht ein solches Mittel mindestens aus einem Träger, an den für humanes IgM spezifische Antikörper gebunden sind, Antigen, markierten antigen-spezifischen Antikörpern, Reagenzien zum Nachweis der Markierung und als Stoff, der verhindert, daß Immunglobulin G an die Festphase und gegebenenfalls Antigen an Immunglobulin G gebunden wird, Anti-Human-IgG, aggregiertes humanes oder tierisches IgG oder ein gamma-Fc-Fragment.

Bevorzugt ist auch ein solches Mittel, das aus einem einzigen Element, in dem alle für das Verfahren erforderlichen Reagenzien in trockener Form enthalten sind, besteht.

Als Trägermaterial für die Festphase sind Kunststoffe wie Polystyrol, Polyvinylchlorid, Polyamid oder andere synthetische Polymere, natürliche Polymere wie Zellulose, sowie derivatisierte natürliche Polymere wie Zelluloseacetat oder Nitrozellulose als auch Glas, insbesondere Glasfasern geeignet.

Die Träger können die Form von Kugeln, Stäben, Röhrchen und Mikrotestplatten haben. Flächenförmige Gebilde wie Streifenpapiere, Plättchen und Membranen sind ebenfalls geeignet. Die Oberfläche der Träger kann für wässrige Lösungen sowohl durchlässig als auch undurchlässig sein.

Bevorzugte Träger sind Mikrotestplatten.

Eine für das erfindungsgemäße Verfahren geeignete Festphase wird hergestellt, indem eine Antikörperpräparation irreversibel an einen Träger gebunden wird. Der Ausdruck "Festphase" wird in der vorliegenden Schrift sowohl für den Träger selbst als auch für den Träger mit daran gebundenem immunchemische Reaktanten gebraucht.

Eine irreversible Bindung im Sinne der Erfindung liegt beispielsweise vor bei

1) einer adsorptiven Bindung, die nicht durch die in dem Verfahren verwendeten Mittel wie markierte Immunreagenzien, Verdünnungs- oder Pufferlösungen gespalten wird,

2) einer über einen immunchemisch (hochaffine Antikörper) oder nicht immunchemisch bindenden Spacer vermittelte bioaffine Bindung, wobei der Spacer aus Biotin und Avidin oder aus anderen Paarungen von Rezeptoren und Liganden bestehen kann,

3) einer kovalenten direkten Bindung oder

4) einer kovalenten, durch einen chemisch bifunktionellen Spacer vermittelten Bindung

Bevorzugt wird die kovalente Bindung im Falle der Verwendung von wasserdurchlässigen Trägern oder die adsorptive Bindung im Falle der Verwendung von wasserdurchlässigen als auch wasserundurchlässigen Trägern.

Besonders bevorzugt wird die direkte adsorptive Bindung von Antikörperpräparationen an mit Gammastrahlen behandeltes Polystyrol als Träger.

Für die Antikörper-Markierung können monoklonale oder polyklonale Antikörper sowie antigen-bindende Fragmente davon verwendet werden, die nach Verfahren gewonnen werden, die als Stand der Technik beschrieben sind.

Als Antigene zur Herstellung markierter Antigene kommen klassisch gereinigte Proteine, synthetische Peptide oder gentechnisch hergestellte Proteine in Frage, deren Darstellung als Stand der Technik beschrieben ist.

Die Markierung erfolgt nach Verfahren, die für die genannten Marker als Stand der Technik beschrieben sind.

Im Falle der Markierung der Antikörper mit Peroxidase als Enzym kann die Periodat Technik (J. Histochem. Cytochem. 1974, 22, 1084-1090) angewandt werden oder ein Verfahren gemäß J. Immunoassay (1983) 4, 209-327, in dem die Partner mit einem heterobifunktionellen Reagenz verknüpft werden.

Die Anwendungsmöglichkeiten der beschriebenen Erfindung eines Einschritt-Immuntests sind grundsätzlich identisch mit den Anwendungen der bereits vorbeschriebenen direkten und indirekten mehrstufigen Tests. Von diesen unterscheidet sich das vorliegende neue Verfahren in dreierlei Hinsicht vorteilhaft:

Durch die Simultan-Inkubation von analyt-haltiger Probe und markierten Immunreagenzien entfällt ein Inkubationsschritt und ein Waschvorgang, was eine erhebliche Vereinfachung der Testdurchführung zur Folge hat.

Wie aus dem zitierten Beispiel ersichtlich wird, gestattet das Einschritt-Verfahren eine erhebliche Verkürzung der Gesamtdauer des Tests, was neben dem vordergründigen Vorteil einer praktikablen Handhabbarkeit des Verfahrens auch in der Klinik für den raschen Nachweis akuter Infektionen eine große Bedeutung besitzt.

Drittens ist es durch dieses Einschritt-Verfahren möglich die Probe bei einer hohen Testverdünnung zu untersuchen, was zur Elimination einer möglichen Kompetition führt und damit eine zuverlässige, reproduzierbare Bestimmung mit gleichzeitiger höherer Nachweisempfindlichkeit von antigen-spezifischen Immunglobulinen erlaubt.

Das folgende Beispiel stellt eine Ausführungsform der Erfindung dar, ohne sie jedoch darauf zu beschränken.

Beispiel

Bestimmung von Toxoplasma-spezifischem Immunglobulin M in humanem Serum

A. Präparation von polyklonalem Anti-Human-IgM

Ziegen-anti-human-IgM wurde hergestellt wie in Methods of Enzymatic Analysis, 3rd Edition 1986, Volume X, Antigens and Antibodies 1, Editor in Chief: Hans Ulrich Bergmeyer, p. 292-308 beschrieben.

B. Herstellung von Toxoplasma Antigen

Toxoplasma gondii Parasiten wurden in der Bauchhöhle von Mäusen über 3 Tage vermehrt. Nach Tötung der Mäuse wurden die Parasiten durch Spülung der Bauchhöhle mit phosphatgepufferter Kochsalzlösung, pH 7.2 gewonnen, durch wiederholte Sedimentation mittels Zentrifugation gewaschen und resuspendiert. Eine so hergestellte Suspension wurde unter Kühlung ultraschallbehandelt, abzentrifugiert und der Überstand als Antigen für die Enzymmarkierung eingesetzt.

C. Enzymmarkierung des Antigens

a) 20 mg Peroxidase (POD) wurden in 0.5 ml phosphatgepufferter Kochsalzlösung (PBS), pH 7.0 aufgenommen und durch Zugabe von 0.6 ml Natriumperjodat aktiviert. Nach ca. 30 min bei Raumtemperatur wurde der Perjodatüberschuß durch Chromatographie (G25 Sephadex) entfernt und das braungrüne Eluat (aktivierte POD) aufgefangen.

b) Kopplung der Peroxidase an das Antigen

Zu zwei Gewichtsteilen aktivierter Peroxidase wurde 1 Gewichtsteil Toxoplasma-Antigen mit Kochsalz-Karbonatpuffer, pH 9.5 gegeben und vermischt. Nach Inkubation für 2 h bei Raumtemperatur, wurden die gebildeten Schiff-Basen durch Zugabe von Natriumborhydrid (1 mg/1 mg POD) reduziert. Das farbige Konjugat wurde durch Zugabe von 1 mg/ml Phenol und 2 % Rinderserumalbumin stabilisiert. Die optimale Gebrauchsverdünnung im Test wurde durch Schachbrettitration ermittelt, wobei toxoplasma-IgM-positive und -negative Seren wie in Abschnitt E beschrieben im Einschritt-Test mit verschiedenen Konzentrationen des Antigen-Peroxidase-Konjugats ausgewertet wurden. Als optimale Konzentration wurde diejenige gewählt, bei der der Signalunterschied zwischen positiver und negativer Probe am größten war.

D. Beschichtung von Polystyrol-Mikrotitrationsplatten mit Anti-Human IgM

Bestrahlte Polystyrol-Mikrotitrationsplatten (gemäß Europäische Patentschrift 0 061 167) wurden pro Vertiefung mit 100 $\mu$l einer Lösung von Anti-Human IgM in phosphatgepufferter Kochsalzlösung, pH 7.5, mehrere Stunden bei Raumtemperatur inkubiert. Die optimale Konzentration der Antikörperlösung wurde vorher mittels serieller Verdünnung und Austestung dieser Probenbeschichtung ermittelt. Anschließend wurden die Platten leergesaugt, mit phosphatgepufferter Kochsalzlösung gewaschen, mittels Silikagel getrocknet und luft- und wasserdampfdicht verpackt.

E. Bestimmung von Toxoplasma IgM-Antikörpern nach dem erfindungsgemäßen Einschrittverfahren

a) Eine Serumprobe sowie je eine Toxoplasma IgM-positive und Toxoplasma IgM-negative Kontrollprobe wurden je 1:350 verdünnt mit 0.3 mol/l Trispufferlösung, pH 7.5, enthaltend 5 ml/100 ml toxoplasmaantikörperfreies Rinderserum, 0.1 ml/100 ml [R]Tween 20 (Polyoxyäthylensorbitanmonolaurat) und Antikörper gegen Human IgG (gamma-Kette) in einer Konzentration, daß 15 mg/ml IgG, bezogen auf die unverdünnte Probe, gebunden werden;

b) jeweils 50 $\mu$l davon wurden in je eine Vertiefung der beschichteten Mikrotitrationsplatten gegeben, in der zuvor pro Vertiefung 50 $\mu$l Peroxidase-markiertes Toxoplasma-Antigen in vorher mittels Schachbrettitration bestimmter optimaler Konzentration, in demselben Puffer vorgelegt worden war;

c) die Testplatte wurde abgedeckt und 2 h bei 37°C inkubiert;

d) anschließend wurde der Inhalt abgesaugt und 3 mal mit PBS, enthaltend 0.1 ml/100 ml [R]Tween 20, gewaschen;

e) nun wurden pro Vertiefung 100 µl Chromogen (o-Phenylendiamin-HCl) in Citrat-Phosphatpuffer, pH 5.5 zugegeben und bei Raumtemperatur für 30 min inkubiert;

f) danach wurden pro Vertiefung 100 µl 1 normale Schwefelsäure zur Beendigung der enzymatischen Substratumsetzung zugegeben und die Lösungen in einem Photometer bei 492 nm gemessen.

g) Beispiele für erhalten Ergebnisse:

| Tabelle 1: | Milliextinktion (mE 492 nm) |
|---|---|
| stark positive Kontrolle | 1080 |
| schwach positive Kontrolle | 314 |
| negative Kontrolle | 58 |
| positive Probe | 1302 |
| negative Probe | 104 |

Eine Probe ist positiv zu werten, wenn sie über einem Wert liegt, der sich aus der Extinktion der Negativ-Kontrolle plus 100 mE (im Beispiel 158 mE) ergibt.

F. Bestimmung von IgM-Antikörpern nach dem Zweischrittverfahren des Standes der Technik

Es wurden das gleiche Peroxidase-markierte Toxoplasma-Antigen verwandt, dessen Herstellung im Abschnitt Cb) des Beispiels, und die gleichen Mikrotestplatten, deren Herstellung im Abschnitt D des Beispiels beschrieben ist.

a) Die in Tabelle 2 aufgeführten Serumproben wurden je 1:200 mit 0.3 mol/l Trispufferlösung, pH 7.5 enthaltend 5 ml/100 ml toxoplasmaantikörperfreies Rinderserum und 0.1 ml/100 ml [R]Tween 20 (Polyoxyäthylensorbitanmonolaurat) verdünnt;

b) jeweils 50 µl davon wurden in je eine Vertiefung der beschichteten Mikrotestplatte gegeben;

c) die Testplatte wurde abgedeckt und 1 h bei 37°C inkubiert;

d) es wurde der Inhalt abgesaugt und 3 mal mit PBS, enthaltend 0.1 ml/100 ml [R]Tween 20 gewaschen;

e) anschließend wurden je Vertiefung 50 µl peroxidasemarkiertes Toxoplasma-Antigen zugegeben;

f) die Testplatte wieder abgedeckt und 2 h bei 37°C inkubiert;

g) danach der Inhalt abgesaugt und 3 mal mit PBS, enthaltend 0.1 ml/100 ml [R]Tween 20 gewaschen;

h) nun wurden pro Vertiefung 100 µl Chromogen (o-Phenylendiamin-HCl) in Citrat-Phosphatpuffer, pH 5.5 zugegeben und bei Raumtemperatur für 30 min inkubiert;

i) danach wurden pro Vertiefung 100 µl 1 normale Schwefelsäure zur Beendigung der enzymatischen Substratumsetzung zugegeben und die Lösungen in einem Photometer bei 492 nm gemessen;

Die in Tabelle 2 aufgeführten Serumproben wurden auch nach dem in den Abschnitten Ea) bis Ef) beschriebenen Einschrittverfahren behandelt.

Die Ergebnisse beider Verfahren sind in Tabelle 2 dargestellt.

Tabelle 2:

| | Milliextinktion (mE $_{492\ nm}$) | |
|---|---|---|
| | Einschrittverfahren | Zweischrittverfahren |
| | Testdauer 2,0 h | Testdauer 3,5 h |
| Pos. Kontrolle | 1.400 (1.600) | 2.300 |
| Neg. Kontrolle | 88 ( 141) | 136 |
| Patientenseren 1 | 204 (145) | 155 |
| 2 | 498 (127) | 133 |
| 3 | 495 (102) | 159 |
| 4 | 499 (110) | 123 |
| 5 | 466 (113) | 118 |
| 6 | 466 (158) | 202 |
| 7* | 1480 (428) | 621 |

( )  Beim Einschrittverfahren erhaltene Extinktionswerte ohne Zusatz von RF-Absorbens

*  Enthält 30 g/l IgG

Aus den gemessenen Werten für die Extinktion, die ein Maß für den Gehalt an Toxoplasma IgM-Antikörpern in den Seren sind, ist zu ersehen, daß das Einschrittverfahren positive Ergebnisse bei den Patientenseren 1-6 liefert ($mE_{492\ nm}$ größer als 88 plus 100), die nach dem Zweischrittverfahren negative Ergebnisse ($mE_{492\ nm}$ kleiner als 136 plus 100) brachten.

Das Weglassen von RF-Absorbens im Einschrittverfahren verursacht bei vielen Seren falsch negative Werte, beispielsweise bei den Seren 1-6, oder erniedrigte Werte beispielsweise bei dem Serum 7, wie aus den Werten in Klammern ersichtlich wird.

Der Nachweis von Toxoplasma IgM-Antikörpern nach dem Einschrittverfahren ist demnach empfindlicher und der Nachweis der genannten und anderer Antikörper schneller und einfacher durchzuführen, wie ein Vergleich des Arbeitsaufwands für beide Verfahren zeigt, der aus den Abschnitten E und F ersichtlich wird.

**Patentansprüche**
**Patentansprüche für folgende Vertragsstaaten : AT, BE, CH, DE, FR, GB, IT, LI, LU, NL, SE**

**1.** Immunchemisches Verfahren zur Bestimmung von Antikörpern, die für ein Antigen spezifisch sind, aus einer der Immunglobulin-Klassen A, M, D oder E in einer Flüssigkeit, wobei eine Festphase, an die Antikörper gegen diese Immunglobulin-Klasse oder ein Fragment eines solchen Antikörpers gebunden sind, mit dieser Flüssigkeit in Kontakt gebracht wird, wodurch das Immunglobulin dieser Klasse an diese Festphase gebunden wird, und diese Festphase entweder mit dem ein Markierungsmittel tragenden Antigen oder mit einem unmarkierten Antigen und einem markierten Antikörper oder einem markierten Fragment eines Antikörpers gegen das Antigen, in Kontakt gebracht wird und aus der Menge des an die Festphase gebundenen Markierungsmittels die Menge dieser Antikörper, die für ein Antigen spezifisch sind, aus einer der Immunglobulin-Klassen bestimmt wird, dadurch gekennzeichnet, daß die Festphase gleichzeitig mit der die zu bestimmenden Antikörper enthaltenden Flüssigkeit und dem entweder markierten oder unmarkierten Antigen in Kontakt ist, wobei als Stoff, der verhindert, daß Immunglobulin G an die Festphase und gegebenenfalls Antigen an Immunglobulin G gebunden wird, Anti-Human-IgG, aggregiertes humanes oder tierisches IgG oder ein gamma-Fc-Fragment zugesetzt

7

wird.

2. Verfahren nach Anspruch 1, dadurch gekennzeichnet, daß markiertes Antigen verwendet wird.

3. Verfahren nach Anspruch 1, dadurch gekennzeichnet, daß unmarkiertes Antigen und ein gegen dieses Antigen gerichteter markierter Antikörper oder ein markiertes Fragment eines solchen Antikörpers verwendet wird.

4. Verfahren nach Anspruch 1, dadurch gekennzeichnet, daß einer oder beide Antikörper monoklonale Antikörper oder ein Fragment monoklonaler Antikörper sind.

5. Verfahren nach Anspruch 1, dadurch gekennzeichnet, daß die zu bestimmenden Antikörper solche der Immunglobulin-Klasse M sind, die gerichtet sind gegen Hepatitis B-core Protein, gegen Antigene von Hepatitis A-, Human Immunodeficiency- (HIV), Röteln- oder Cytomegalie-Virus oder Proteine von Treponema pallidum oder Toxoplasma gondii.

6. Verfahren nach Anspruch 1, dadurch gekennzeichnet, daß das Markierungsmittel ein Enzym ist.

7. Verfahren nach Anspruch 1, dadurch gekennzeichnet, daß das Markierungsmittel Erythrozyten sind.

8. Mittel zur Ausführung des Verfahrens nach Anspruch 1 mindestens bestehend aus einem Träger, an den für humanes IgM spezifische Antikörper gebunden sind, sowie markiertem Antigen, für das dieses IgM spezifisch ist, Reagenzien zum Nachweis oder zur Bestimmung der Markierung, und als Stoff, der verhindert, daß Immunglobulin G an die Festphase und gegebenenfalls Antigen an Immunglobulin G gebunden wird, Anti-Human-IgG, aggregiertes humanes oder tierisches IgG oder ein gamma-Fc-Fragment.

9. Mittel zur Ausführung des Verfahrens nach Anspruch 1, mindestens bestehend aus einem Träger, an den für humanes IgM spezifische Antikörper gebunden sind, sowie Antigen, markiertem antigen-spezifischen Antikörper, Reagenzien zum Nachweis der Markierung und als Stoff, der verhindert, daß Immunglobulin G an die Festphase und gegebenenfalls Antigen an Immunglobulin G gebunden wird, Anti-Human-IgG, aggregiertes humanes oder tierisches IgG oder ein gamma-Fc-Fragment.

10. Mittel zur Ausführung des Verfahrens nach Anspruch 1, bestehend aus einem einzigen Element, in dem alle für das Verfahren erforderlichen Reagenzien in trockener Form enthalten sind.

**Patentansprüche für folgenden Vertragsstaat : ES**

1. Immunchemisches Verfahren zur Bestimmung von Antikörpern, die für ein Antigen spezifisch sind, aus einer der Immunglobulin-Klassen A, M, D oder E in einer Flüssigkeit, wobei eine Festphase, an die Antikörper gegen diese Immunglobulin-Klasse oder ein Fragment eines solchen Antikörpers gebunden sind, mit dieser Flüssigkeit in Kontakt gebracht wird, wodurch das Immunglobulin dieser Klasse an diese Festphase gebunden wird, und diese Festphase entweder mit dem ein Markierungsmittel tragenden Antigen oder mit einem unmarkierten Antigen und einem markierten Antikörper oder einem markierten Fragment eines Antikörpers gegen das Antigen, in Kontakt gebracht wird und aus der Menge des an die Festphase gebundenen Markierungsmittels die Menge dieser Antikörper, die für ein Antigen spezifisch sind, aus einer der Immunglobulin-Klassen bestimmt wird, dadurch gekennzeichnet, daß die Festphase gleichzeitig mit der die zu bestimmenden Antikörper enthaltenden Flüssigkeit und dem entweder markierten oder unmarkierten Antigen in Kontakt ist, wobei als Stoff, der verhindert, daß Immunglobulin G an die Festphase und gegebenenfalls Antigen an Immunglobulin G gebunden wird, Anti-Human-IgG, aggregiertes humanes oder tierisches IgG oder ein gamma-Fc-Fragment zugesetzt wird.

2. Verfahren nach Anspruch 1, dadurch gekennzeichnet, daß markiertes Antigen verwendet wird.

3. Verfahren nach Anspruch 1, dadurch gekennzeichnet, daß unmarkiertes Antigen und ein gegen dieses Antigen gerichteter markierter Antikörper oder ein markiertes Fragment eines solchen Antikörpers verwendet wird.

**4.** Verfahren nach Anspruch 1, dadurch gekennzeichnet, daß einer oder beide Antikörper monoklonale Antikörper oder ein Fragment monoklonaler Antikörpers sind.

**5.** Verfahren nach Anspruch 1, dadurch gekennzeichnet, daß die zu bestimmenden Antikörper solche der Immunglobulin-Klasse M sind, die gerichtet sind gegen Hepatitis B-core Protein, gegen Antigene von Hepatitis A-, Human Immunodeficiency- (HIV), Röteln- oder Cytomegalie-Virus oder Proteine von Treponema pallidum oder Toxoplasma gondii.

**6.** Verfahren nach Anspruch 1, dadurch gekennzeichnet, daß das Markierungsmittel ein Enzym ist.

**7.** Verfahren nach Anspruch 1, dadurch gekennzeichnet, daß das Markierungsmittel Erythrozyten sind.

**Claims**
**Claims for the following Contracting States : AT, BE, CH, DE, FR GB, IT, LI, LU, NL, SE**

**1.** An immunochemical method for the determination of antibodies which are specific for an antigen and are of one of the immunoglobulin classes A, M, D or E in a fluid, entailing this fluid being contacted with a solid phase to which the antibodies against this immunoglobulin class, or a fragment of an antibody of this type, are bound, which results in the immunoglobulin of this class being bound to this solid phase, and this solid phase being contacted either with the antigen which carries a labeling means or with an unlabeled antigen and with a labeled antibody or a labeled fragment of an antibody against the antigen, and determination, from the amount of labeling means which is bound to the solid phase, of the amount of these antibodies which are specific for an antigen and are of one of the immunoglobulin classes, which comprises the solid phase being simultaneously in contact with the fluid containing the antibodies which are to be determined and with the antigen, which is either labeled or unlabeled, there being addition of anti-human IgG, aggregated human or animal IgG or a gamma-Fc fragment, as substance which prevents immunoglobulin G binding to the solid phase and, where appropriate, antigen binding to immunoglobulin G.

**2.** The method as claimed in claim 1, wherein labeled antigen is used.

**3.** The method as claimed in claim 1, wherein unlabeled antigen and a labeled antibody which is directed against this antigen, or a labeled fragment of an antibody of this type, is used.

**4.** The method as claimed in claim 1, wherein one or both antibodies are monoclonal antibodies or a fragment of monoclonal antibodies.

**5.** The method as claimed in claim 1, wherein the antibodies which are to be determined are those of immunoglobulin class M directed against hepatitis B core protein, against antigens of hepatitis A virus, human immunodeficiency virus (HIV), rubella virus or cytomegalovirus, or proteins of Treponema pallidum or Toxoplasma gondii.

**6.** The method as claimed in claim 1, wherein the labeling means is an enzyme.

**7.** The method as claimed in claim 1, wherein the labeling means are erythrocytes.

**8.** An agent for carrying out the method as claimed in claim 1, composed at the least of a carrier to which antibodies specific for human IgM are bound, and of labeled antigen for which this IgM is specific, reagents for the detection or for the determination of the labeling, and anti-human IgG, aggregated human or animal IgG or a gamma-Fc fragment, as substance which prevents immunoglobulin G binding to the solid phase and, where appropriate, antigen binding to immunoglobulin G.

**9.** An agent for carrying out the method as claimed in claim 1, composed at the least of a carrier to which antibodies specific for human IgM are bound, and of antigen, of labeled antigen-specific antibody, of reagents for the detection of the labeling, and anti-human IgG, aggregated human or animal IgG or a gamma-Fc fragment, as substance which prevents immunoglobulin G binding to the solid phase and, where appropriate, antigen binding to immunoglobulin G.

**10.** An agent for carrying out the method as claimed in claim 1, composed of a single element which contains, in dry form, all the reagents required for the method.

**Claims for the following Contracting State : ES**

**1.** An immunochemical method for the determination of antibodies which are specific for an antigen and are of one of the immunoglobulin classes A, M, D or E in a fluid, entailing this fluid being contacted with a solid phase to which the antibodies against this immunoglobulin class, or a fragment of an antibody of this type, are bound, which results in the immunoglobulin of this class being bound to this solid phase, and this solid phase being contacted either with the antigen which carries a labeling means or with an unlabeled antigen and with a labeled antibody or a labeled fragment of an antibody against the antigen, and determination, from the amount of labeling means which is bound to the solid phase, of the amount of these antibodies which are specific for an antigen and are of one of the immunoglobulin classes, which comprises the solid phase being simultaneously in contact with the fluid containing the antibodies which are to be determined and with the antigen, which is either labeled or unlabeled, there being addition of anti-human IgG, aggregated human or animal IgG or a gamma-Fc fragment, as substance which prevents immunoglobulin G binding to the solid phase and, where appropriate, antigen binding to immunoglobulin G.

**2.** The method as claimed in claim 1, wherein labeled antigen is used.

**3.** The method as claimed in claim 1, wherein unlabeled antigen and a labeled antibody which is directed against this antigen, or a labeled fragment of an antibody of this type, is used.

**4.** The method as claimed in claim 1, wherein one or both antibodies are monoclonal antibodies or a fragment of monoclonal antibodies.

**5.** The method as claimed in claim 1, wherein the antibodies which are to be determined are those of immunoglobulin class M directed against hepatitis B core protein, against antigens of hepatitis A virus, human immunodeficiency virus (HIV), rubella virus or cytomegalovirus, or proteins of Treponema pallidum or Toxoplasma gondii.

**6.** The method as claimed in claim 1, wherein the labeling means is an enzyme.

**7.** The method as claimed in claim 1, wherein the labeling means are erythrocytes.

**Revendications**
**Revendications pour les Etats contractants suivants : AT, BE, CH, DE, FR, GB, IT, LI, LU, NL, SE**

**1.** Procédé immunochimique pour doser des anticorps, spécifiques d'un antigène, appartenant à l'une des classes d'immunoglobulines A, M, D ou E, dans un liquide, consistant à mettre une phase solide, à laquelle sont liés des anticorps dirigés contre cette classe d'immunoglobulines, ou un fragment de tels anticorps, en contact avec ce liquide, grâce à quoi l'immunoglobuline de cette classe se lie à la phase solide, et à mettre la phase solide en contact avec l'antigène portant un marqueur ou avec un antigène non marqué et un anticorps marqué ou un fragment marqué d'un anticorps dirigé contre l'antigène, et à déterminer la quantité de cet anticorps, spécifique d'un antigène, appartenant à l'une des classes d'immunoglobulines, d'après la quantité du marqueur qui s'est lié à la phase solide, caractérisé en ce que la phase solide est en contact, simultanément, avec le liquide contenant les anticorps à doser et avec l'antigène, marqué ou non, une IgG anti-humaine, une IgG agrégée, humaine ou animale, ou un fragment de gamma-Fc étant ajoutés en tant que substance destinée à empêcher que l'immunoglobuline G se lie à la phase solide et, éventuellement, que l'antigène se lie à l'immunoglobuline G.

**2.** Procédé selon la revendication 1, caractérisé en ce que l'on utilise un antigène marqué.

**3.** Procédé selon la revendication 1, caractérisé en ce que l'on utilise un antigène non marqué et un anticorps marqué, dirigé contre cet antigène, ou un fragment marqué d'un tel anticorps.

**4.** Procédé selon la revendication 1, caractérisé en ce qu'un anticorps ou les deux anticorps sont des anticorps monoclonaux ou un fragment d'anticorps monoclonal.

**5.** Procédé selon la revendication 1, caractérisé en ce que les anticorps à doser sont ceux, appartenant à la classe des immunoglobulines M, qui sont dirigés contre la protéine centrale de l'hépatite B, contre les antigènes des virus de l'hépatite A, de l'immunodéficience humaine (VIH), de la rougeole ou les antigènes du cytomégalovirus, ou contre les protéines de Treponema pallidum ou de Toxoplasma gondii.

**6.** Procédé selon la revendication 1, caractérisé en ce que le marqueur est une enzyme.

**7.** Procédé selon la revendication 1, caractérisé en ce que le marqueur est constitué d'érythrocytes.

**8.** Agent pour effectuer le procédé selon la revendication 1, constitué au moins d'un support auquel sont liés des anticorps spécifiques de l'IgM humaine, ainsi que d'un antigène marqué, spécifique de cette IgM, de réactifs pour caractériser ou doser le marqueur, et d'une IgG anti-humaine, une IgG agrégée, humaine ou animale, ou un fragment de gamma-Fc en tant que substance destinée à empêcher que l'immunoglobuline G se lie à la phase solide et, éventuellement, que l'antigène se lie à l'immunoglobuline G.

**9.** Agent pour effectuer le procédé selon la revendication 1, constitué au moins d'un support auquel sont liés des anticorps spécifiques de l'IgM humaine, ainsi que d'un antigène, d'un anticorps marqué, spécifique de l'antigène, de réactifs pour caractériser ou doser le marqueur, et d'une IgG anti-humaine, une IgG agrégée, humaine ou animale, ou un fragment de gamma-Fc, en tant que substance destinée à empêcher que l'immunoglobuline G se lie à la phase solide et, éventuellement, que l'antigène se lie à l'immunoglobuline G.

**10.** Agent pour effectuer le procédé selon la revendication 1, constitué d'un élément unique qui contient tous les réactifs nécessaires au procédé, sous forme déshydratée.

**Revendications pour l'Etat contractant suivant : ES**

**1.** Procédé immunochimique pour doser des anticorps, spécifiques d'un antigène, appartenant à l'une des classes d'immunoglobulines A, M, D ou E, dans un liquide, consistant à mettre une phase solide, à laquelle sont liés des anticorps dirigés contre cette classe d'immunoglobulines, ou un fragment de tels anticorps, en contact avec ce liquide, grâce à quoi l'immunoglobuline de cette classe se lie à la phase solide, et à mettre la phase solide en contact avec l'antigène portant un marqueur ou avec un antigène non marqué et un anticorps marqué ou un fragment marqué d'un anticorps dirigé contre l'antigène, et à déterminer la quantité de cet anticorps, spécifique d'un antigène, appartenant à l'une des classes d'immunoglobulines, d'après la quantité du marqueur qui s'est lié à la phase solide, caractérisé en ce que la phase solide est en contact, simultanément, avec le liquide contenant les anticorps à doser et avec l'antigène, marqué ou non, une IgG anti-humaine, une IgG agrégée, humaine ou animale, ou un fragment de gamma-Fc étant ajoutés en tant que substance destinée à empêcher que l'immunoglobuline G se lie à la phase solide et, éventuellement, que l'antigène se lie à l'immunoglobuline G.

**2.** Procédé selon la revendication 1, caractérisé en ce que l'on utilise un antigène marqué.

**3.** Procédé selon la revendication 1, caractérisé en ce que l'on utilise un antigène non marqué et un anticorps marqué, dirigé contre cet antigène, ou un fragment marqué d'un tel anticorps.

**4.** Procédé selon la revendication 1, caractérisé en ce qu'un anticorps ou les deux anticorps sont des anticorps monoclonaux ou un fragment d'anticorps monoclonal.

**5.** Procédé selon la revendication 1, caractérisé en ce que les anticorps à doser sont ceux, appartenant à la classe des immunoglobulines M, qui sont dirigés contre la protéine centrale de l'hépatite B, contre les antigènes des virus de l'hépatite A, de l'immunodéficience humaine (VIH), de la rougeole ou les antigènes du cytomégalovirus, ou contre les protéines de Treponema pallidum ou de Toxoplasma gondii.

**6.** Procédé selon la revendication 1, caractérisé en ce que le marqueur est une enzyme.

**7.** Procédé selon la revendication 1, caractérisé en ce que le marqueur est constitué d'érythrocytes.